# EUROPÄISCHE PATENTSCHRIFT

(11) **EP 3 518 989 B1**
(45) Veröffentlichungstag und Bekanntmachung des Hinweises auf die Patenterteilung: **03.09.2025**
(21) Anmeldenummer: 17786834.6
(22) Anmeldetag: 29.09.2017
(51) Int. Cl.: A61L 2/20, B01B 1/00

(54) **VORRICHTUNG ZUM VERDAMPFEN EINES FLÜSSIGEN MEDIUMS IN EINER FÜLLPRODUKTABFÜLLANLAGE**
DEVICE FOR EVAPORATING A LIQUID MEDIUM IN A FILLING PRODUCT FILLING SYSTEM
DISPOSITIF D'ÉVAPORATION D'UN MILIEU LIQUIDE DANS UNE INSTALLATION DE MISE EN BOUTEILLE D'UN PRODUIT DE REMPLISSAGE

(30) Priorität: 29.09.2016 DE 102016118475
(43) Veröffentlichungstag der Anmeldung: 07.08.2019
(73) Patentinhaber: Krones AG, 93073 Neutraubling (DE)
(72) Erfinder: PETER, Michael, 93073 Neutraubling (DE); SOELLNER, Juergen, 93073 Neutraubling (DE)
(74) Vertreter: Nordmeyer, Philipp Werner
(86) Internationale Anmeldenummer: PCT/EP2017/074772
(87) Internationale Veröffentlichungsnummer: WO 2018/060422

(56) Entgegenhaltungen:
- EP-A1- 2 407 181
- EP-A1- 2 407 181
- EP-A2- 2 604 294
- EP-A2- 2 604 294
- WO-A1-2010/086375
- WO-A2-2006/125417
- WO-A2-2006/125417
- CN-U- 205 145 623
- DE-A1- 3 540 161
- DE-C- 391 744
- GB-A- 987 121
- JP-A- 2005 065 882
- JP-A- H09 294 805
- US-A1- 2004 050 503
- US-A1- 2004 050 503

## Beschreibung

### Technisches Gebiet

Die vorliegende Erfindung betrifft eine Vorrichtung zum Verdampfen eines flüssigen Mediums in einer Füllproduktabfüllanlage, beispielsweise zum Verdampfen eines Desinfektionsmediums in einer Getränkeabfüllanlage zum Bereitstellen eines desinfizierenden Gases zur Desinfektion von füllproduktberührten Bereichen der Getränkeabfüllanlage. Die vorliegende Vorrichtung dient beispielsweise zum Verdampfen von Wasserstoffperoxid zur Verwendung in einem Desinfektionsvorgang in einer Getränkeabfüllanlage.

### Stand der Technik

Zum Verdampfen flüssiger Medien sind unterschiedliche Verdampfer und Verdampferformen bekannt, welche beispielsweise auch in Getränkeabfüllanlagen dazu verwendet werden, gasförmiges Wasserstoffperoxid zur Desinfektion von produktberührten Anlagenkomponenten bereitzustellen. Die Verdampfer weisen dabei üblicherweise eine beheizte Verdampferoberfläche auf, auf welche das noch flüssige und zu verdampfende Medium aufgesprüht wird und auf welcher das flüssige Medium dann verdampft wird. Die Verdampferoberfläche wird dabei beispielsweise mittels Heißdampf oder mittels elektrischer Heizstäbe geheizt, um die gewünschte Temperatur der Verdampferoberfläche bereitzustellen, welche entsprechend oberhalb der Siedetemperatur des flüssigen, zu verdampfenden Mediums liegt.

Beispielsweise ist aus der JP 2005 065 882 A2 ein Verdampfer zum Verdampfen einer wässrigen Wasserstoffperoxidlösung bekannt, welche auf eine geneigte Verdampferoberfläche des Verdampfers aufgesprüht wird, um entsprechend eine Verdampfung der Waserstoffperoxidlösung zu ermöglichen. Die Verdampferoberfläche ist in einer Verdampferkammer angeordnet, welche durch ein die Verdampferoberfläche gegenüber der Umgebung abschließendes, kastenförmiges Gehäuse definiert wird.

Die WO 2006/125417 A2 beschreibt einen Totalverdampfer und eine Vorrichtung zur kontrollierten Vermischung, Verdampfung und/oder Reaktion mehrerer Fluide. Die EP 2 604 294 A2 und EP 2 407 181 A1 beschreiben Verdampfer zum Sterilisieren von Behältern. Die GB 987,121 A beschreibt ein Verfahren zum Verdampfen von Flüssigkeiten. Weitere für diese Erfindung relevante Verdampfer werden in den Patentveröffentlichungen CN205145623 U und WO2010/086375 A1 beschrieben.

### Darstellung der Erfindung

Ausgehend von dem bekannten Stand der Technik ist es eine Aufgabe der vorliegenden Erfindung, eine Vorrichtung zum Verdampfen eines flüssigen Mediums anzugeben, welche eine hohe Verdampferleistung bei einem zuverlässigen Aufbau der Vorrichtung ermöglicht.

Diese Aufgabe wird durch eine Vorrichtung mit den Merkmalen des Anspruchs 1 gelöst. Vorteilhafte Weiterbildungen ergeben sich aus den Unteransprüchen sowie aus den Figuren und der vorliegenden Beschreibung.

Entsprechend wird eine Vorrichtung zum Verdampfen eines flüssigen Mediums in einer Füllproduktabfüllanlage vorgeschlagen, umfassend eine geneigte Verdampferoberfläche, einen Medienzulauf zum Aufbringen des zu verdampfenden, flüssigen Mediums auf die Verdampferoberfläche, sowie Rillen in der Verdampferoberfläche zum Leiten des flüssigen Mediums. Erfindungsgemäß sind die Rillen entlang der Verdampferoberfläche nichtlinear ausgebildet.

Dadurch, dass die Rillen, welche zum Leiten des flüssigen, zu verdampfenden Mediums in der Verdampferoberfläche vorgesehen sind, nichtlinear ausgebildet sind, kann das zu verdampfende, flüssige Medium entlang dieser Rillen auf der Verdampferoberfläche verlangsamt fließen, so dass eine gleichmäßige Verteilung des flüssigen, zu verdampfenden Mediums auf der Verdampferoberfläche bereitgestellt werden kann. Entsprechend kann die Verdampferoberfläche mit dem flüssigen, zu verdampfenden Medium über die gesamte Verdampferoberfläche hinweg gleichmäßig beaufschlagt werden, so dass die Verdampferleistung insgesamt erhöht werden kann, da die aktive Verdampferoberfläche vergrößert ist.

Durch die Ausbildung der Rillen ist es weiterhin auch nicht notwendig, Sprühdüsen zu verwenden, welche das flüssige, zu verdampfende Medium auf die Verdampferoberfläche aufbringen, sondern das Medium kann über den Medienzulauf einfach auf die Verdampferoberfläche aufgebracht werden und es wird mittels der Rillen gleichmäßig verteilt. Durch die Abwesenheit von Sprühdüsen kann es auch nicht zu einer Verstopfung der Sprühdüsen kommen, so dass eine besonders zuverlässige und wartungsarme Vorrichtung angegeben werden kann.

Unter einer nicht-linearen Ausbildung der Rillen wird insbesondere verstanden, dass sich die Rillen in der jeweiligen Verdampferoberfläche nicht über ihre gesamte Länge hinweg gerade erstrecken, sondern eine Richtungsänderung erfahren. Dies kann beispielsweise durch mit Bogen versehene Rillen, spiralförmige Rillen, im Zick-Zack mit abrupten Richtungswechseln versehene Rillen und andere Rillenformen erreicht werden, welche sich nicht gerade auf der jeweiligen Verdampferoberfläche erstrecken. Die Rillen können sich insbesondere auch spiralförmig oder bogenförmig oder gebogen entlang der Verdampferoberfläche erstrecken.

Durch die Ausgestaltung der Rillenform kann entsprechend erreicht werden, dass das zu verdampfende, flüssige Medium in gelenkter Form auf der Verdampferoberfläche verteilt werden kann, so dass eine weitgehend vollständige Benetzung der Verdampferoberfläche mit dem zu verdampfenden Medium gewährleistet ist und damit eine hohe Verdampferleistung erreicht wird.

Vorzugsweise ist die Verdampferoberfläche in einem Verdampfergehäuse aufgenommen, wobei die Kontur der Innenwand des Verdampfergehäuses zumindest im Bereich der geneigten Verdampferoberfläche im Wesentlichen der Kontur der Verdampferoberfläche folgt.

Die Verdampferoberfläche und das Verdampfergehäuse bilden damit entsprechend eine Verdampferkammer aus, in welcher das Volumen, in dem die Verdampfung stattfindet, durch den Abstand zwischen Verdampferoberfläche und der Innenseite des Verdampfergehäuses ausgebildet wird. Durch die parallele Führung der Oberflächen zueinander wird eine kanalförmige Struktur mit einem im Wesentlichen konstanten Abstand ausgebildet. Die Verdampferkammer ist dabei entsprechend zumindest in den Bereichen, in welchen das Verdampfergehäuse der Verdampferoberfläche gegenüberliegt und dessen Kontur folgt, im Querschnitt im Wesentlichen ringförmig ausgebildet.

Auf diese Weise kann das Volumen der Verdampferkammer kontrolliert werden beziehungsweise so klein gehalten werden, dass eine Ausgestaltung der Verdampferkammer und damit das Volumen der Verdampferkammer klein bleibt. Dies ist insbesondere im Hinblick auf die Regelungen der Druckgeräterichtlinie 97/23/EG von Bedeutung, da Druckbehälter nicht abnahmepflichtig sind, wenn das Druckvolumenprodukt, also das Produkt aus dem Volumen der Druckkammer mal der Druckstufe des Behälters, kleiner als 50 ist. Kann das Volumen der Verdampferkammer klein gehalten werden, kann auf diese Weise ein wartungsarmer und nicht-abnahmepflichtiger Verdampfer bereitgestellt werden, welcher sowohl aus Kostengründen als auch aus Gründen der Anlageneffizienz vorteilhaft ist.

Die Verdampferoberfläche ist durch einen kegelförmigen oder pyramidenförmigen oder konoiden Verdampferkörper bereitgestellt. Der Verdampferkörper kann dabei insbesondere auch als Kegelstumpf ausgebildet sein, auf dessen Oberseite eine Verteilung des aus dem Medienzulauf fließenden, flüssigen, zu verdampfenden Mediums an die einzelnen Rillen sowie auf die gesamte Verdampferoberfläche erreicht wird.

Folgt die Innenkontur des Verdampfergehäuses der Verdampferoberfläche, so entsteht entsprechend nur ein ringförmiger Spalt beziehungsweise ein kegelmantelförmiger Spalt zwischen der Verdampferoberfläche und der Innenseite des Verdampfergehäuses, entlang dessen das Trägergas beziehungsweise die Trägerluft an der Verdampferoberfläche entlang streichen kann. Selbst bei höheren Drücken, welche durch die Verdampfung des flüssigen Mediums auftreten, kann damit weiterhin das Druckvolumenprodukt so kontrolliert werden, dass innerhalb des Verdampfers eine Überschreitung der in der Druckgeräterichtlinie vorgegebenen Grenzwerte nicht auftritt und entsprechend eine effiziente und wartungsarme Vorrichtung bereit gestellt werden kann.

Bevorzugt schließt sich an den oberen Bereich der Verdampferoberfläche nach oben hin eine Medienverteileinrichtung an, die bevorzugt domförmig ausgebildet ist. Besonders bevorzugt weist die Medienverteileinrichtung einen Dom auf, auf den das flüssige Medium von dem Medienzulauf aufgebracht wird. In einer Weiterbildung ist zwischen dem Dom und der Verdampferoberfläche eine Rinne zum Aufnehmen von flüssigem Medium angeordnet und besonders bevorzugt stehen die sich entlang der Verdampferoberfläche erstreckenden Rillen mit der Rinne in Fluidverbindung.

Im Falle, dass der Verdampferkörper kegelförmig beziehungsweise kegelstumpfförmig ist, sind die Rillen, die sich entlang der Verdampferoberfläche erstrecken, in einer entlang der Kegelachse blickenden Projektion bevorzugt spiralförmig ausgebildet.

Bevorzugt ist eine Medienzufuhr vorgesehen, welche jeweils in dem am höchsten liegenden Bereich der geneigten Verdampferoberfläche ein Zuführen des flüssigen, zu verdampfenden Mediums auf die geneigte Verdampferoberfläche ermöglicht. Die Medienzufuhr kann dabei, aufgrund der Rillen, ohne die Verwendung von Sprühdüsen erfolgen, da die Verteilung des flüssigen, zu verdampfenden Mediums durch die nichtlinearen Rillen über die Verdampferoberfläche hinweg vorgenommen wird.

Dadurch, dass auf ein Aufsprühen des Mediums auf die Verdampferoberfläche verzichtet werden kann, können auch Störungen, welche sich durch das Verstopfen von Sprühdüsen, so wie es aus den herkömmlichen Verdampfern bekannt ist, ergeben, vermieden werden. Vielmehr kann über eine Medienzufuhr beispielsweise in Form eines Rohrs, welches einen vorgegebenen Querschnitt hat, das zu verdampfende, flüssige Medium auf den obersten Punkt beziehungsweise auf den obersten Bereich der Verdampferoberfläche ohne eine Veränderung des Querschnitts des Rohrs der Medienzufuhr aufgebracht werden.

Das Rohr für die Medienzufuhr wird dabei bevorzugt in seinem Querschnitt so gewählt, dass das flüssige, zu verdampfende Medium konstant in einer Menge zugeführt werden kann, welche auch von der Vorrichtung direkt verdampft werden kann. Mit anderen Worten wird das zugeführte Medium sofort verdampft und die Medienzufuhr kann kontinuierlich betrieben werden.

In die Verdampferkammer wird ein Trägergas über eine Trägergaszufuhr zugeführt und dann bevorzugt über eine Gasableitung zum Ableiten des mit dem verdampften Medium angereicherten Trägergases aus der Verdampferkammer wieder abgeleitet.

In einer bevorzugten Ausführungsform ist die Trägergaszufuhr so ausgeprägt, dass sie das Trägergas mit einem Impuls auf die Verdampferoberfläche richtet, um auf diese Weise weiterhin ein effizientes Verdampfen zu unterstützen. Durch das Umströmen beziehungsweise Überströmen der Verdampferoberfläche mit dem Trägergas kann ein effizienter Abtransport des verdampften flüssigen Mediums zusammen mit dem Trägergas erreicht werden.

Die Trägergaszufuhr kann dabei bevorzugt über eine Düse so in die Verdampferkammer eingeblasen werden, dass eine turbulente, verwirbelte Strömung entlang der Verdampferoberfläche erzeugt wird, um einen effizienten Abtransport des verdampften Mediums zu erreichen.

Die Trägergaszufuhr umfasst einen Trägergaskanal, welcher um den Umfang der kegelförmigen und/oder pyramidenförmigen und/oder konoiden Verdampferoberfläche herum angeordnet ist und über Trägergasauslässe das Trägergas auf die Verdampferoberfläche ausgibt. Damit kann ein gleichmäßiges Überstreichen der Verdampfungsoberfläche mit dem Trägergas erreicht werden, so dass eine effiziente Ausnutzung der gesamten Verdampferoberfläche erreicht wird.

Bevorzugt wird in einer weiteren Ausbildung der Vorrichtung zum Verdampfen das Einblasen des Trägergases beziehungsweise der Luft, welche mit dem verdampften flüssigen Medium angereichert werden soll, über eine Mehrzahl von Zuführöffnungen beziehungsweise einen Zuführschlitz durchgeführt, um entsprechend eine im Wesentlichen gleichmäßige Beaufschlagung der Verdampferoberfläche mit dem Trägergas zu erreichen. Auch auf diese Weise kann die Effizienz der Verdampfung erhöht werden und gleichzeitig für einen zuverlässigen und gleichmäßigen Abtransport des verdampften flüssigen Mediums gesorgt werden.

Die Ausbildung der Gasströmung entlang der Verdampferoberfläche ist bevorzugt turbulent, um den Abtransport der Verdampfungsprodukte beziehungsweise die Aufnahme des verdampften flüssigen Mediums in dem Trägergas zu unterstützen. Aus Gründen der Energieeffizienz kann die Strömung jedoch auch als laminare Strömung angelegt werden, um die innerhalb der Verdampfungskammer vorliegenden Widerstände zu reduzieren.

In einer weiteren bevorzugten Ausbildung ist eine Heizvorrichtung für die Verdampferoberfläche vorgesehen, welche zum Betrieb mit einem Heizmedium eingerichtet ist, welches eine Siedetemperatur aufweist, die oberhalb der Arbeitstemperatur der Verdampferoberfläche liegt. Durch diese Auslegung der Heizvorrichtung kann eine Beheizung stattfinden, die keinen weiteren sich aus der Druckgeräterichtlinie ergebenden Beschränkungen unterliegt. Als effizientes Heizmedium kann beispielsweise ein Thermoöl verwendet werden, das mit einem geringen Druck dem Verdampferkörper zugeführt wird.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine schematische Schnittdarstellung durch eine Vorrichtung zum Verdampfen eines flüssigen Mediums in Form einer wässrigen H₂O₂ Lösung;
- Figur 2: eine teilgeschnittene, perspektivische Darstellung der Vorrichtung aus Figur 1;
- Figur 3: eine schematische, geschnittene Darstellung der Vorrichtung aus den Figuren 1 und 2, in welcher der durch die Verdampferkammer streichende Luftstrom schematisch angedeutet ist; und
- Figur 4: eine schematische, teilgeschnittene, perspektivische Darstellung einer Vorrichtung zum Verdampfen eines flüssigen Mediums in einer weiteren Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf die wiederholte Beschreibung dieser Elemente in der nachfolgenden Beschreibung teilweise verzichtet.

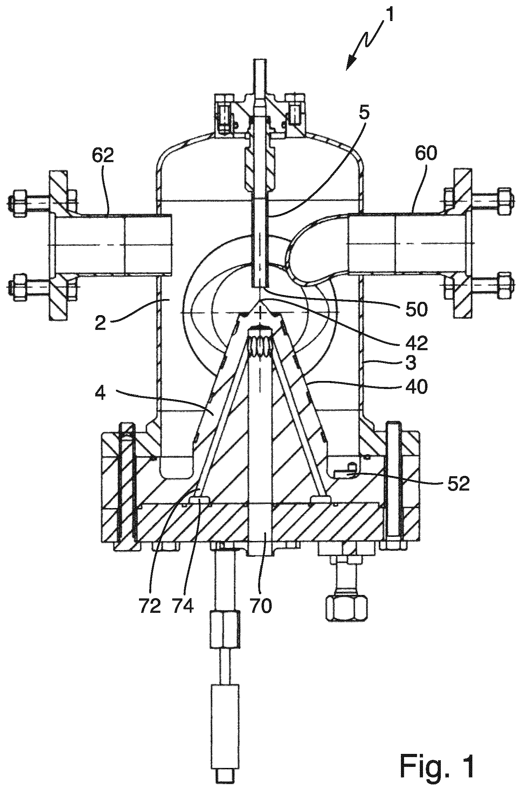

In den Figuren 1 bis 3 ist eine schematische Darstellung einer Vorrichtung 1 zum Verdampfen eines flüssigen Mediums dargestellt. Dabei wird als flüssiges Medium beispielsweise eine wässrige 35%-ige H₂O₂-Lösung in der Vorrichtung 1 verdampft und damit ein Trägergas, beispielsweise Luft, angereichert. Das mit dem verdampften flüssigen Medium angereicherte Trägergas wird dann nachfolgend beispielsweise in einem Desinfektionsprozess in einer Füllproduktabfüllanlage verwendet, um diese beispielsweise turnusgemäß zu desinfizieren oder aber um diese nach einer Umrüstung oder einem Anlagenstillstand wieder in einen hygienisch einwandfreien Zustand zu bringen, damit bei der nachfolgenden Abfüllung des Füllprodukts, beispielsweise eines Getränks oder eines anderen Lebensmittels, eine hygienisch einwandfreie Abfüllung bereitgestellt werden kann.

Die Vorrichtung 1 zum Verdampfen des flüssigen Mediums umfasst eine Verdampferkammer 2, welche durch ein Verdampfergehäuse 3 definiert ist. In dem Verdampfergehäuse 3 und mithin in der Verdampferkammer 2 ist ein Verdampferkörper 4 vorgesehen, der eine Verdampferoberfläche 40 ausbildet. Die Verdampferoberfläche 40 ist geneigt orientiert ausgebildet, so dass das aufgebrachte flüssige Medium im Prinzip nach unten hin abfließt.

Auf die Verdampferoberfläche 40 des Verdampferkörpers 4 wird über ein Medienzulauf 5 das zu verdampfende, flüssige Medium aufgebracht und entsprechend auf der Verdampferoberfläche 40 verdampft, so dass das verdampfte Medium in die Verdampferkammer 2 gelangt.

Um das verdampfte Medium, das auf der Verdampferoberfläche 40 verdampft wurde und sich in der Verdampferkammer 2 befindet, abtransportieren zu können und nachfolgend in einer Füllproduktabfüllanlage beispielsweise zur Desinfektion verwenden zu können, wird über eine Trägergaszufuhr 60 ein Trägergas in die Verdampfungskammer 2 eingeleitet und dann über einen Gasauslass 62 gemeinsam mit dem auf der Verdampferoberfläche 40 verdampften Medium abgeleitet. Der Gasauslass 62 dient entsprechend dazu, das verdampfte Medium gemeinsam mit dem Trägergas an einen nachfolgenden Bearbeitungsschritt zu übergeben. Das Trägergas kann beispielsweise Luft sein.

Der Verdampferkörper 4 ist in dem gezeigten Ausführungsbeispiel im Wesentlichen kegelförmig ausgestaltet. Entsprechend ist die Verdampferoberfläche 40 in Form einer Kegeloberfläche ausgestaltet. Die kegelförmige Ausbildung des Ventilkörpers 4 ermöglicht es, auf einer relativ kleinen Grundfläche eine relativ große Verdampferoberfläche 40 bereitzustellen und dabei eine einfach reinigbare Vorrichtung 1 ohne Hinterschnitte bereit zu stellen.

Um das über den Medienzulauf 5, welcher in dem gezeigten Ausführungsbeispiel in Form eines Zuführrohres ausgebildet ist, durch welches die H₂O₂-Lösung auf die Verdampferoberfläche 40 gebracht wird, zugeführte flüssige Medium gleichmäßig auf die Verdampferoberfläche 40 aufzubringen, ist im oberen Bereich des Verdampferkörpers 4 ein Dom 42 bereitgestellt, welcher das aus dem Medienzulauf 5 ausfließende Medium gleichmäßig um den Umfang der Verdampferoberfläche 40 herum verteilt.

Dazu kann zwischen dem Dom 42 und der Verdampferoberfläche 40 eine Rinne angeordnet sein, welche das flüssige Medium bis zu ihrem Überlaufen zurückhält und welche zu einer gleichmäßigen Verteilung des flüssigen Mediums auf der Verdampferoberfläche 40 führt und insbesondere eine gleichmäßige Verteilung um den Umfang herum ermöglicht.

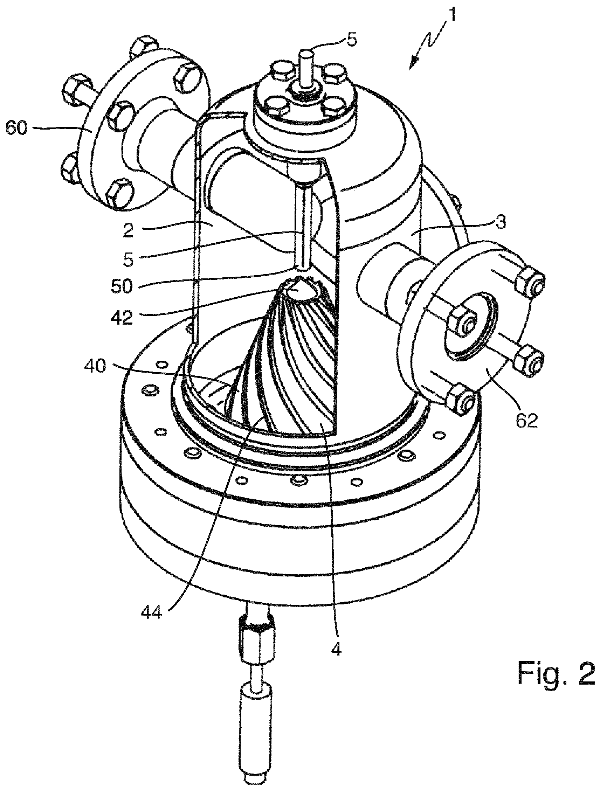

Zusätzlich sind in der Verdampferoberfläche 40 Rillen 44 vorgesehen, entlang welcher das zu verdampfende Medium geführt wird und entsprechend über die Verdampferoberfläche 40 verteilt werden kann. Die Rillen 44, welche in der Verdampferoberfläche 40 des Verdampferkörpers 4 angeordnet sind, sind, wie beispielsweise aus Figur 2 besonders gut zu erkennen, auf der Verdampferoberfläche nichtlinear beziehungsweise krumm oder gebogen angeordnet. Die Rillen 44 stehen besonders bevorzugt mit der Rinne in Fluidverbindung und entspringen in einer bevorzugten Weiterbildung in der Rinne.

In der speziellen Ausführung der Figuren 1 bis 3 sind die Rillen 44 in spiralförmigen Abschnitten auf der Verdampferoberfläche 40 angeordnet. Durch die nichtlineare und besonders die spiralförmige Anordnung der Rillen 44 wird ein effizienter Transport des flüssigen, noch nicht verdampften Mediums über die gesamte Verdampferoberfläche 40 hinweg ermöglicht und gleichzeitig wird durch die spiralförmige Ausbildung verhindert, dass das flüssige, noch nicht verdampfte Medium sofort nach unten abfließt und sich nur im unteren Bereich ansammelt. Vielmehr kann über die nichtlineare beziehungsweise spiralförmige Ausbildung der Rillen 44 erreicht werden, dass das flüssige, noch nicht verdampfte, aber noch zu verdampfende Medium langsamer fließt und es sich gleichmäßig über die Verdampferoberfläche 40 verteilt. Entsprechend kann eine besonders effiziente Verdampfung des zu verdampfenden, flüssigen Mediums erreicht werden, da sämtliche Oberflächenbereiche der Verdampferoberfläche 40 gleichmäßig und kontinuierlich mit dem flüssigen Medium beaufschlagt werden können.

Die Rillen 44 erstrecken sich dabei besonders bevorzugt von dem obersten Bereich der Verdampferoberfläche 40 bis in deren untersten Bereich, so dass eine gleichmäßige Verteilung des flüssigen Mediums über die gesamte Verdampferoberfläche 40 hinweg erreicht werden kann.

Der Medienzulauf 5 ist in dem gezeigten Ausführungsbeispiel in Form eines Rohres ausgebildet, welches an seinem Austrittsende 50 keinerlei Verjüngung oder Düse aufweist. Mit anderen Worten fließt das zu verdampfende, flüssige Medium direkt auf den Dom 42 des Verdampferkörpers 4 und wird erst durch den Dom 42 auf die Verdampferoberfläche 40 verteilt. Damit ist der Medienzulauf 5 nicht mit einer Düse zum Versprühen des zu verdampfenden Mediums ausgestattet, so dass ein zuverlässiger Betrieb und eine Verringerung der Erfordernisse an die Wartung des Medienzulaufs 5 innerhalb der Vorrichtung 1 dadurch erreicht wird, dass ein Zusetzen, Verstopfen oder Neueinstellen von Düsen zur Zufuhr des zu verdampfenden Mediums nicht notwendig ist.

Der Verdampferkörper 4 weist einen Heizkreislauf auf, welcher einn Heizmedienzulauf 70 in Form einer zentralen Bohrung innerhalb des kegelförmigen Verdampferkörpers 4 bereitstellt. Ein Heizmedienrücklauf 72 ist in Form einer Mehrzahl sich radial ausgehend von einem oberen Bereich des Heizmedienzulaufs 70 erstreckenden, parallel zur Verdampferoberfläche 40 geführten Bohrungen in dem Verdampferkörper 4 vorgesehen. Entsprechend kann das Heizmedium über den Heizmedienzulauf 70 zentral in dem Verdampferkörper 4 hochgeführt werden, und dann über den Heizmedienrücklauf 72 entlang der Verdampferoberfläche 40 innerhalb des Verdampferkörpers 4 zurückgeführt werden, so dass eine gleichmäßige und effiziente Wärmeübertragung auf die Verdampferoberfläche 40 innerhalb des Verdampferkörpers 4 möglich ist. Der Heizmedienrücklauf 72 in Form der sich parallel zu der Verdampferoberfläche 40 erstreckenden Bohrungen mündet am unteren Ende des Verdampferkörpers 4 in einen Rückfuhrkanal 74, mittels welchem das Heizmedium dann wieder an einen externen Wärmetauscher zurückgeführt werden kann.

Als Heizmedium wird bevorzugt ein solches verwendet, welches eine Siedetemperatur aufweist, die oberhalb der Arbeitstemperatur der Vorrichtung 1 beziehungsweise oberhalb der gewünschten Arbeitstemperatur der Verdampferoberfläche 40 liegt. In dem gezeigten Ausführungsbeispiel wird hierfür bevorzugt ein Thermoöl verwendet, dessen Siedetemperatur deutlich oberhalb der Arbeitstemperatur der Verdampferoberfläche 40 liegt. Auf diese Weise kann der Heizkreislauf mit dem Heizmedienzulauf 70 und dem Heizmedienrücklauf 72 sowie dem Rückführkanal 74 bei einem geringen Druck betrieben werden und das Auftreten von Druckspitzen durch Verdampfen des Heizmediums innerhalb des Heizkreislaufs kann vermieden werden. Auf diese Weise wird eine Beheizung der Verdampferoberfläche 40 besonders sicher bereitgestellt.

Alternativ zu der beschriebenen Beheizung des Verdampferkörpers 4 über ein Thermoöl oder ein anderes wärmetragendes Medium mit einer Siedetemperatur, die oberhalb der Arbeitstemperatur der Verdampferoberfläche 40 liegt, kann der Verdampferkörper 4 und insbesondere die Verdampferoberfläche 40 auch mit elektrischen Heizstäben oder auf eine andere bekannte Art und Weise beheizt werden, um gleichzeitig eine hohe Betriebssicherheit bereitstellen zu können und andererseits eine zuverlässige und gleichmäßige Beheizung der Verdampferoberfläche 40 zu erreichen.

Das über den Medienzulauf 5 und insbesondere das Austrittsende 50 des Medienzulaufs 5 zugeführte, flüssige Medium, welches auf der Verdampferoberfläche 40 nicht verdampft wird, kann periodisch oder bedarfsgesteuert über einen Medienauslass 52, welcher im unteren Bereich der Verdampferkammer 2 angeordnet ist, aus der Verdampferkammer 2 abgelassen und entfernt werden.

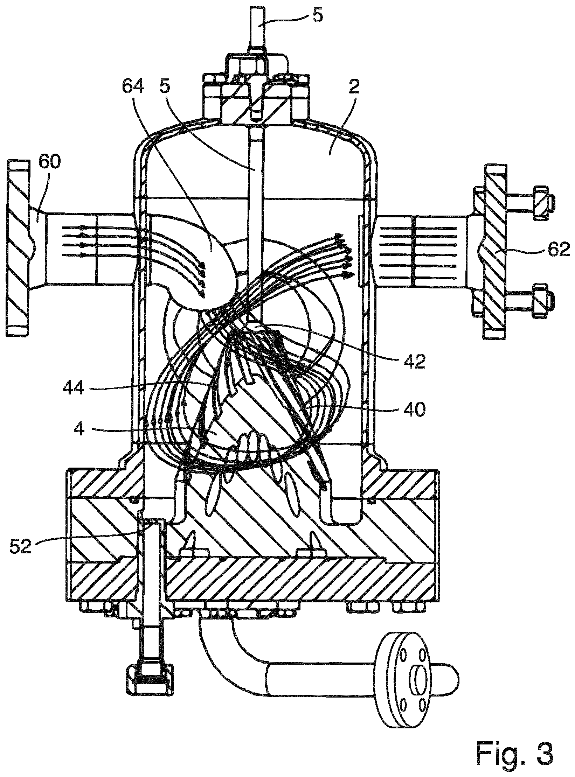

Wie sich beispielsweise aus der Darstellung der Figur 3 ergibt, wird das Trägergas über die Trägergaszufuhr 60 in die Verdampferkammer 2 eingeblasen. Die Trägergaszufuhr 60 weist dabei einen Umlenkbereich 64 auf, mittels welchem das Trägergas direkt auf die Verdampferoberfläche 40 des Verdampferkörpers 4 aufgeblasen werden kann, so wie beispielsweise durch die schematisch angedeuteten Pfeile innerhalb der Verdampferkammer 2 in Figur 3 angedeutet. Auf diese Weise kann erreicht werden, dass der Trägergasstrom, welcher über die Trägergaszufuhr 60 zugeführt wird, in einer verwirbelten und turbulenten Strömung auf die Verdampferoberfläche 40 auftrifft und auf diese Weise einen effizienten Abtransport des verdampften flüssigen Mediums ermöglicht.

Das Auftreten der turbulenten Strömung wird durch die Rillen 44, welche in der Verdampferoberfläche 40 des Verdampferkörpers 4 vorgesehen sind, weiter unterstützt, so dass aufgrund der vielschichtigen und vielfältigen Verwirbelung auch an den Rillen 44 ein besonders effizienter Abtransport der Verdampfungsprodukte erreicht wird.

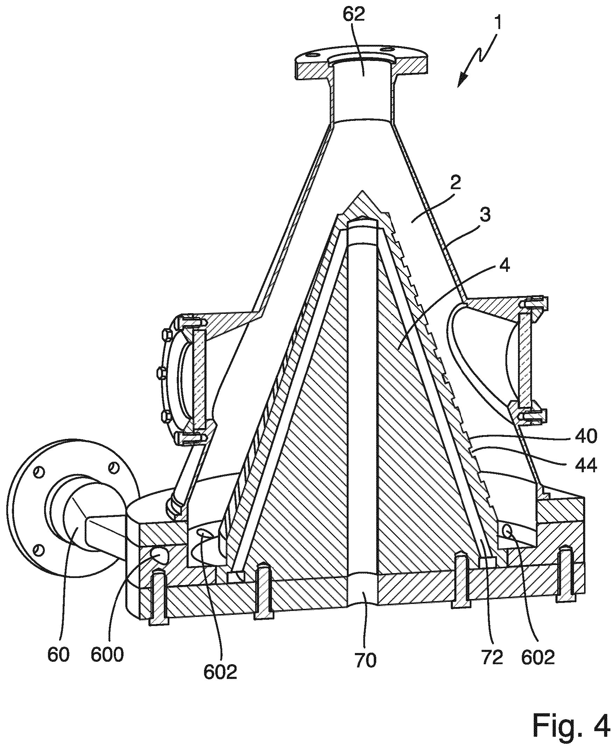

In einer alternativen Ausführungsform, welche in einer schematischen, perspektivischen und teilgeschnittenen Darstellung in Figur 4 gezeigt ist, ist die Vorrichtung 1 zum Verdampfen eines flüssigen Mediums wiederum mit einer Verdampferkammer 2 versehen, welche durch ein Verdampfergehäuse 3 ausgebildet wird, in welchem ein kegelförmiger Verdampferkörper 4 vorgesehen ist. Die Ausbildung des Verdampferkörpers 4 mit seiner kegelförmigen Ausbildung und entsprechend der kegelmantelförmigen Verdampferoberfläche 40 entspricht im Wesentlichen der aus den vorher besprochenen Ausführungsformen.

In dem Ausführungsbespiel der Figur 4 ist das Verdampfergehäuse 3 jedoch so ausgebildet, dass die Kontur der Innenseite des Verdampfergehäuses 3 im Wesentlichen der Kontur der Verdampferoberfläche 40 des Verdampferkörpers 4 folgt. Entsprechend ist auch das Verdampfergehäuse 3 im Wesentlichen kegelförmig ausgebildet, so dass der sich zwischen der Verdampferoberfläche 40 und der Innenseite des Verdampfergehäuses 3 ausbildende Raum der Verdampferkammer 2 entsprechend im Querschnitt einem ringförmigen Kanal entspricht. Die Verdampferoberfläche 40 und die Innenseite des Verdampfergehäuses 30 sind entsprechend im Wesentlichen parallel zueinander geführt.

Auf diese Weise kann erreicht werden, dass das Volumen der Verdampferkammer 2, welches durch die Innenwand des Verdampfergehäuses 3 definiert wird und durch die Volumenverdrängung des Verdampferkörpers 4 reduziert wird, so kontrolliert werden kann, dass die Verdampferkammer 2 nur ein relativ kleines Volumen ausbildet. Entsprechend kann, auch auf Grundlage der Druckgeräterichtlinie 97/23/EG, das Druckvolumenprodukt der Vorrichtung 1 zum Verdampfen des flüssigen Mediums so klein gehalten werden, dass der Druckbehälter nach der Druckgeräterichtlinie nicht abnahmepflichtig wird.

Unter dem Druckvolumenprodukt oder dem Druckinhaltsprodukt wird die sich aus der Multiplikation des Volumens der Druckkammer mit der Druckstufe des Behälters ergebende Zahl verstanden, welche gemäß der Druckgeräterichtlinie kleiner als 50 bar*I sein muss, um zu erreichen, dass der Behälter nicht abnahmepflichtig ist. Dies wird beispielsweise bei einem Volumen der Verdampferkammer 2 von 8 Litern bei einem Arbeitsdruck von 6 bar erreicht, wobei dann ein Druckvolumenprodukt von beispielsweise 48 bar*I erreicht wird.

Entsprechend kann durch die Dimensionierung des Verdampferkörpers 4 und der geometrischen Gestaltung des den Verdampferkörper 4 umgebenden Verdampfergehäuses 3 derart, dass die Verdampferkammer 2 ein möglichst kleines Volumen aufweist, erreicht werden, dass der Verdampfer trotz der Auslegung für höhere Drücke aufgrund des geringen Druckvolumenprodukts nicht abnahmepflichtig ist und entsprechend die Installations- und Wartungskosten sowie der Zeitaufwand reduziert werden können.

Die Luftführung des Trägergases ist in dem Ausführungsbeispiel der Figur 4 so gelöst, dass eine Trägergaszufuhr 60 vorgesehen ist, welche das Trägergas in einen sich unten um den Umfang des Verdampfergehäuses 3 herum erstreckenden Trägergaskanal 600 einbläst, und um den Umfang des Verdampferkörpers 4, welcher hier in Form eines Kegels ausgebildet ist, unterschiedliche Trägergasauslässe 602 vorgesehen sind, welche ein Einströmen des Trägergases in die Verdampferkammer 2 ermöglichen. Entsprechend wird das Trägergas, welches über die Trägergaszufuhr 60 zugeführt ist und über den Trägergaskanal 600 um den Umfang der Verdampferkammer 2 in deren Bodenbereich herum verteilt wird, über die Trägergasauslässe 602 in die Verdampferkammer 2 eingeblasen. Entsprechend findet eine gleichmäßige Zufuhr des Trägergases in die Verdampferkammer 2 statt, so dass das Trägergas entlang der Verdampferoberfläche 40 des Verdampferkörpers 4 streichen kann, um dann, gemeinsam mit den Verdampfungsprodukten, aus dem Gasauslass 62 ausgelassen zu werden.

Durch die kegelförmige Verjüngung der Verdampferkammer 2 findet in dem gezeigten Ausführungsbeispiel nach oben hin - also zum Gasauslass 62 hin - eine Verringerung des Querschnitts der Verdampferkammer 2 statt, welche in einer Erhöhung der Fließgeschwindigkeit des Trägergases innerhalb der Verdampfungskammer 2 resultiert. Dies korreliert mit der aufgrund der Führung des Heizmedienstroms in der Heizmedienzulauf 70 und den Heizmedienrückläufe 72 vorgegebenen Temperaturverteilung der Verdampferoberfläche 40 derart, dass die Verdampferoberfläche 40 im oberen Bereich etwas wärmer ist, als in deren unterem Bereich. Weiterhin reduziert sich die Verdampferoberfläche 40 in deren oberen Bereich, so dass aufgrund der höheren Luftströmungsgeschwindigkeit und der höheren Temperatur hier dennoch eine hohe Verdampfungsleistung aufrechterhalten werden kann.

Ist ein solcher Druckverlauf und Verlauf der Fließgeschwindigkeit des Trägergases innerhalb der Verdampferkammer 2 nicht gewünscht, so kann anstelle der Ausbildung des Verdampfergehäuses 3 mit einer exakt gleichen Innenwandneigung wie der Verdampferoberfläche 40 auch eine Verdampferkammer 2 ausgebildet werden, bei welcher sich das Verdampfergehäuse 3 in Richtung der Kegelspitze des Verdampferkörpers 4 nach außen hin öffnet, um einen gleichbleibenden Strömungsquerschnitt über die gesamte Verdampferkammer 2 hinweg bereitzustellen.

### Bezugszeichenliste

- 1: Vorrichtung zum Verdampfen eines flüssigen Mediums
- 2: Verdampferkammer
- 3: Verdampfergehäuse
- 4: Verdampferkörper
- 40: Verdampferoberfläche
- 42: Dom
- 44: Rillen
- 5: Medienzulauf
- 50: Austrittsende
- 52: Medienauslass
- 60: Trägergaszufuhr
- 62: Gasauslass
- 64: Umlenkbereich
- 600: Trägergaskanal
- 602: Trägergasauslass
- 70: Heizmedienzulauf
- 72: Heizmedienrücklauf
- 74: Rückführkanal

## Patentansprüche

1. Vorrichtung (1) zum Verdampfen eines flüssigen Mediums in einer Füllproduktabfüllanlage, umfassend eine geneigte Verdampferoberfläche (40), einen Medienzulauf (5) zum Aufbringen des zu verdampfenden, flüssigen Mediums auf die Verdampferoberfläche (40), sowie Rillen (44) in der Verdampferoberfläche (40) zum Leiten des flüssigen Mediums, wobei
die Verdampferoberfläche (40) kegelförmig und/oder pyramidenförmig und/oder konoid ausgebildet ist und die Rillen (44) entlang der Verdampferoberfläche (40) nichtlinear ausgebildet sind,
**dadurch gekennzeichnet, dass**
eine Trägergaszufuhr (60) vorgesehen ist, welche ein Trägergas auf die Verdampferoberfläche (40) leitet, wobei die Trägergaszufuhr (60) einen Trägergaskanal (600) umfasst, welcher um den Umfang der Verdampferoberfläche (40) herum angeordnet ist und über Trägergasauslässe (602) das Trägergas auf die Verdampferoberfläche (40) ausgibt.

2. Vorrichtung (1) gemäß Anspruch 1, **dadurch gekennzeichnet, dass** sich die Rillen (44) spiralförmig oder bogenförmig oder gebogen entlang der Verdampferoberfläche (40) erstrecken.

3. Vorrichtung (1) gemäß Anspruch 1 oder 2, **dadurch gekennzeichnet, dass** die Verdampferoberfläche (40) in einem Verdampfergehäuse (3) aufgenommen ist und die Kontur der Innenwand des Verdampfergehäuses (3) zumindest im Bereich der geneigten Verdampferoberfläche (40) im Wesentlichen der Kontur der Verdampferoberfläche (40) folgt.

4. Vorrichtung (1) gemäß einem der vorstehenden Ansprüche, **dadurch gekennzeichnet, dass** sich an den oberen Bereich der Verdampferoberfläche (40) nach oben hin eine Medienverteileinrichtung anschließt, die bevorzugt domförmig ausgebildet ist.

5. Vorrichtung (1) gemäß Anspruch **4, dadurch gekennzeichnet, dass** die Medienverteileinrichtung einen Dom (42) aufweist, auf den das flüssige Medium von dem Medienzulauf (5) aufgebracht wird.

6. Vorrichtung (1) gemäß Anspruch 5, **dadurch gekennzeichnet, dass** zwischen dem Dom (42) und der Verdampferoberfläche (40) eine Rinne zum Aufnehmen von flüssigem Medium angeordnet ist und bevorzugt die sich entlang der Verdampferoberfläche (40) erstreckenden Rillen (44) mit der Rinne in Fluidverbindung stehen.

7. Vorrichtung (1) gemäß einem der vorstehenden Ansprüche, **dadurch gekennzeichnet, dass** eine Heizvorrichtung für die Verdampferoberfläche (40) vorgesehen ist, welche zum Betrieb mit einem Heizmedium eingerichtet ist, welches eine Siedetemperatur aufweist, die oberhalb der Arbeitstemperatur der Verdampferoberfläche (40) liegt.

8. Vorrichtung (1) gemäß einem der vorstehenden Ansprüche, **dadurch gekennzeichnet, dass** der Medienzulauf (5) durch ein Rohr konstanten Querschnitts ausgebildet ist.

## Claims

1. Device (1) for evaporating a fluid medium in a filling product filling plant, comprising an inclined evaporator surface (40), a medium supply line (5) for applying the fluid medium that is to be evaporated to the evaporator surface (40), and grooves (44) in the evaporator surface (40) for conducting the fluid medium, wherein
the evaporator surface (40) is cone-shaped and/or pyramid-shaped and/or conoid-shaped and the grooves (44) along the evaporator surface (40) have a non-linear design,
**characterized in that**
a carrier gas supply (60) is provided which directs a carrier gas onto the evaporator surface (40), wherein the carrier gas supply (60) comprises a carrier gas channel (600) which is arranged around the circumference of the evaporator surface (40) and discharges the carrier gas onto the evaporator surface (40) via carrier gas outlets (602).

2. Device (1) according to claim 1, **characterized in that** the grooves (44) extend along the evaporator surface (40) in a spiral and/or curved and/or bent form.

3. Device (1) according to claim 1 or 2, **characterized in that** the evaporator surface (40) is accommodated in an evaporator housing (3), and the contour of the inner wall of the evaporator housing (3), at least in the region of the inclined evaporator surface (40), substantially follows the contour of the evaporator surface (40).

4. Device (1) according to one of the previous claims, **characterized in that** the upper region of the evaporator surface (40) is adjoined in an upwards direction by a medium distribution device, which preferably has a dome-shaped design.

5. Device (1) according to claim 4, **characterized in that** the medium distribution device has a dome (42), onto which the fluid medium is applied by the medium supply line (5).

6. Device (1) according to claim 5, **characterized in that** a gutter for accommodating fluid medium is disposed between the dome (42) and the evaporator surface (40), and preferably the grooves (44) which extend along the evaporator surface (40) are in fluid communication with the gutter.

7. Device (1) according to one of the previous claims, **characterized in that** a heating device for the evaporator surface (40) is provided, which is configured to operate with a heating medium that has a boiling temperature above the working temperature of the evaporator surface (40).

8. Device (1) according to one of the previous claims, **characterized in that** the medium supply line (5) is formed by a tube with a constant cross-section.

## Revendications

1. Dispositif (1) pour l'évaporation d'un milieu liquide dans une installation de remplissage à produit de remplissage, comprenant une surface d'évaporation (40) inclinée, une arrivée de milieu (5) pour l'application du milieu liquide à évaporer sur la surface d'évaporation (40), ainsi que des cannelures (44) dans la surface d'évaporation (40) pour le guidage du milieu liquide, dans lequel
la surface d'évaporation (40) est réalisée en forme conique et/ou pyramidale et/ou conoïde et les cannelures (44) sont réalisées de manière non linéaire le long de la surface d'évaporation (40),
**caractérisé en ce que**
une alimentation en gaz porteur (60) est prévue, laquelle guide un gaz porteur sur la surface d'évaporation (40), dans lequel l'alimentation en gaz porteur (60) comprend un canal de gaz porteur (600) qui est disposé autour de la circonférence de la surface d'évaporation (40) et qui délivre le gaz porteur sur la surface d'évaporation (40) par l'intermédiaire de sorties de gaz porteur (602).

2. Dispositif (1) selon la revendication 1, **caractérisé en ce que** les cannelures (44) s'étendent en forme de spirale ou d'arc ou de manière courbée le long de la surface d'évaporation (40).

3. Dispositif (1) selon la revendication 1 ou 2, **caractérisé en ce que** la surface d'évaporation (40) est logée dans un boîtier d'évaporation (3) et le contour de la paroi intérieure du boîtier d'évaporation (3) suit sensiblement le contour de la surface d'évaporation (40) au moins dans la zone de la surface d'évaporation (40) inclinée.

4. Dispositif (1) selon l'une des revendications précédentes,
**caractérisé en ce qu'un** appareil de distribution de milieu, de préférence réalisé en forme de dôme, se raccorde vers le haut à la zone supérieure de la surface d'évaporation (40).

5. Dispositif (1) selon la revendication 4, **caractérisé en ce que** l'appareil de distribution de milieu présente un dôme (42) sur lequel le milieu liquide est appliqué depuis l'arrivée de milieu (5).

6. Dispositif (1) selon la revendication 5, **caractérisé en ce que,** entre le dôme (42) et la surface d'évaporation (40), une rigole est disposée pour la réception d'un milieu liquide et, de préférence, les cannelures (44) s'étendant le long de la surface d'évaporation (40) sont en liaison fluidique avec la rigole.

7. Dispositif (1) selon l'une des revendications précédentes,
**caractérisé ce qu'un** dispositif de chauffage est prévu pour la surface d'évaporation (40), lequel est conçu pour fonctionner avec un milieu chauffant qui présente une température d'ébullition qui est supérieure à la température de travail de la surface d'évaporation (40).

8. Dispositif (1) selon l'une des revendications précédentes,
**caractérisé en ce que** l'arrivée de milieu (5) est réalisée par un tube de section transversale constante.
